# EUROPÄISCHE PATENTSCHRIFT

(11) **EP 0 937 252 B1**
(45) Veröffentlichungstag und Bekanntmachung des Hinweises auf die Patenterteilung: **30.05.2001**
(21) Anmeldenummer: 97947698.3
(22) Anmeldetag: 22.10.1997
(51) Int. Cl.: G01N 33/53

(54) **VERFAHREN ZUM HERKUNFTSNACHWEIS VON NUTZTIEREN SOWIE DAVON STAMMENDEN PRODUKTEN**
METHOD OF ESTABLISHING THE ORIGIN OF USEFUL ANIMALS AND PRODUCTS PRODUCED THEREFROM
PROCEDE PERMETTANT D'ETABLIR L'ORIGINE D'ANIMAUX UTILES ET PRODUITS QUI EN SONT ISSUS

(30) Priorität: 23.10.1996 DE 19643682
(43) Veröffentlichungstag der Anmeldung: 25.08.1999
(73) Patentinhaber: Gareis, Manfred, 95349 Thurnau (DE); Groschup, Martin, 72072 Tübingen (DE)
(72) Erfinder: Gareis, Manfred, 95349 Thurnau (DE); Groschup, Martin, 72072 Tübingen (DE)
(74) Vertreter: Gassner, Wolfgang, Dr.
(86) Internationale Anmeldenummer: DE9702439
(87) Internationale Veröffentlichungsnummer: WO9818003

(56) Entgegenhaltungen:
- EP-A- 0 336 800
- WO-A-95/06723
- GB-A- 2 271 848
- US-A- 4 152 412
- DATABASE WPI Section Ch, Week 9708 Derwent Publications Ltd., London, GB; Class B04, AN 97-080960 XP002059374 & JP 08 322 427 A (FUJITA GAKUEN) , 10.Dezember 1996
- PATENT ABSTRACTS OF JAPAN vol. 000, no. 00 & JP 09 229932 A (KOLLAGEN GIJUTSU), 5.September 1997,
- AGRICOLA DATABASE ABSTRACT 85:9020 "Marking new Lines of Brown Swiss Cattle with alleles of B-system of blood groups." Gurkovich, K.A. und Chernushenko, V.K. Biulleten' nauchnykh rabot - Vsesoiuznyi n.issledovatel'skii institut,1981 S. 81-85 XP002059373

## Beschreibung

Die Erfindung betrifft ein Verfahren und eine Verwendung zum Herkunftsnachweis von Nutztieren, insbesondere von Rindern, Schweinen u. dgl., sowie davon stammenden Produkten.

Nach dem Stand der Technik werden Nutztiere, wie Rinder und Schweine, zum Herkunftsnachweis mit einer Kennung versehen, die in eine am Ohr des Nutztiers befestigte Kennmarke eingeprägt ist (Tätowierung, Transponder u.a. Systeme). Vor dem Schlachten werden die Nutztiere in Abhängigkeit ihrer Herkunft in Gruppen zusammengefaßt. Das Fleisch der geschlachteten Tiere wird sodann mit einem gruppenspezifischen Stempel versehen. - Neuerdings ist man zur Erhöhung der Zuverlässigkeit des Herkunftsnachweises dazu übergegangen, zusätzlich zu jedem Nutztier Begleitpapiere zu erstellen.

Dennoch sind die nach dem Stand der Technik derzeit verwendeten Herkunftsnachweise in vielerlei Hinsicht nachteilig. So ist es Gang und Gebe, daß zum Unterlaufen von Importbeschränkungen oder -sperren die Kennmarken ausgetauscht oder gefälscht und die entsprechenden Begleitpapiere ebenfalls gefälscht werden. Das Erstellen von Begleitpapieren verursacht einen hohen bürokratischen Aufwand.

Gerade zur zuverlässigen Eindämmung des illegalen Handelns mit z.B. potentiell BSE-Erreger kontaminierten Rindfleisch bzw. dessen Verwendung zur Herstellung von Fleischprodukten, wie Hundefutter u.ä., ist der nach dem Stand der Technik verwendete Herkunftsnachweis unzureichend.

Aus der GB 2 271 848 ist es bekannt, Tiere durch die Gabe von Isotopen zu markieren, die natürlicherweise im betreffenden Tier vorkommen. Zur Identifikation wird die Abweichung des Verhältnisses des zugegebenen Isotops gegenüber anderen im Tier natürlicherweise vorkommenden Isotopen ermittelt. Ein solcher Nachweis ist aufwendig.

Aufgabe der Erfindung ist es, die Nachteile nach dem Stand der Technik zu beseitigen. Insbesondere soll ein fälschungssicherer Nachweis bereitgestellt werden, mit dem die Herkunft sowohl des lebenden Nutztiers als auch davon stammender Produkte, wie Fleisch, Fleischerzeugnisse und Lebensmittel tierischer Herkunft, zuverlässig nachweisbar sind.

Diese Aufgabe wird durch die Merkmale der Ansprüche 1 und 14 gelöst. Zweckmäßige Weiterbildungen ergeben sich aus den Merkmalen der Ansprüche 2 bis 13 bzw. 15 bis 23.

Zur Lösung der Aufgabe ist ein Verfahren vorgesehen, welches die folgenden Schritte umfaßt:
a) Biologische Markierung des lebenden Nutztiers durch Applikation mindestens eines für das Nutztier und den Menschen unschädlichen Immunogens, das weder als Impfstoff, noch als Arzneimittel, noch über die Nahrungskette, noch über die Umwelt bei der üblichen Nutztierhaltung in den Organismus des Nutztiers gelangt und gegen das im Nutztier natürlicherweise keine Antikörper gebildet werden, und
b) Nachweis der durch die Applikation von mindestens einem Immunogen gebildeten spezifischen Antikörper im lebenden Nutztier oder in davon stammenden Produkten mittels eines enzymimmunologischen oder immunochemischen Nachweisverfahrens.

Das erfindungsgemäße Verfahren hat den Vorteil, daß die biologische Markierung untrennbar mit dem Nutztier verbunden und daher nicht ausgetauscht oder gefälscht werden kann. Außerdem ist es möglich, die Herkunft von Fleisch ohne Herkunftsstempel, sogar von zur Herstellung von Fleischprodukten verwendetem Fleisch nachzuweisen.

Vorteilhafterweise wird/werden als Immunogen(e) ein Protein und/oder Peptid und/oder ein synthetisch hergestelltes Fragment von Aminosäureteilsequenzen natürlich vorkommender Immunogene verwendet. Dabei wird zweckmäßigerweise ein mit einem Trägerstoff gekoppeltes Protein und/oder Peptid verwendet. Damit kann auf einfache Weise eine spezifische Markierung erzielt werden, die leicht nachweisbar und für den menschlichen Verzehr völlig unbedenklich ist.

Nach einem Ausgestaltungsmerkmal ist das Immunogen derart beschaffen, daß die gebildeten spezifischen Antikörper dauerhaft im Organismus des Nutztiers verbleiben und mittels eines enzymimmunologischen oder immunochemischen Nachweisverfahrens nachweisbar sind. - Auf diese Weise kann die Markierung durch eine einzige Applikation durchgeführt werden, die zweckmäßigerweise unmittelbar nach der Niederkunft des Nutztiers erfolgt. Die Applikation kann erfolgen, indem das Immunogen dem Nutztier durch Injektion appliziert wird. Daneben kann das Immunogen auch über die Schleimhaut, vorzugsweise die Nasenschleimhaut, des Nutztiers, bsp. mit einem Nasenspray, oder mittels eines Implantats appliziert werden.

Vorteilhafterweise wird eine Mischung von mehreren verschiedenen Immunogenen appliziert. Dabei kann ein erstes Immunogen zur Kennzeichnung der Herkunftsregion, wie Westeuropa, Osteuropa, Südamerika usw., ein zweites Immunogen zur Kennzeichnung des Herkunftsstaats und ein drittes Immunogen zur Kennzeichnung von Verwaltungseinheiten des betreffenden Staats verwendet werden. Alternativ oder ergänzend können auch einzelne Produktions- und Vertriebsorganisationen (z.B. Erzeugerringe, Qualitätsfleischprogramme) eine biologische Markierung ihrer Nutztierbestände bzw. davon stammender Produkte anwenden.

Zum Nachweis der Antikörper können beim lebenden Nutztier das Blut, die Milch oder andere Körpersekrete, beim geschlachteten Tier ebenfalls Blut und Körpersekrete, wie der Tropf- oder Pressaft des Fleisches, verwendet werden.

Zur Identifikation der Antikörper wird als enzymimmunologisches oder immunochemisches vorzugsweise eines oder mehrere der folgenden Nachweisverfahren angewendet: Enzyme Linked Immunosorbent Assay (= ELISA), Enzyme Immuno Assay (= EIA) und Radio Immuno Assay (= RIA). Besonders einfach ist der enzymimmunologische oder immunochemische Nachweis, wenn er unter Verwendung von Teststäbchen durchgeführt wird. - Wenn die Markierung unter staatlicher Aufsicht stehenden Tierärzten vorbehalten bleibt, kann die Herkunft von bsp. aus geschlachteten Nutztieren gewonnenen Produkten praktisch durch jedermann geprüft und festgestellt werden.

Nach Maßgabe der Erfindung ist des weiteren die Verwendung mindestens eines
a) für Nutztiere und den Menschen unschädlichen Immunogens zur biologischen Markierung des lebenden Nutztiers sowie davon stammender Produkte, wobei das Immunogen weder als Impfstoff, noch als Arzneimittel, noch über die Nahrungskette bei der üblichen Nutztierhaltung in den Organismus des Nutztiers gelangt und gegen das im Nutztier natürlicherweise keine Antikörper gebildet werden, und
b) eines enzymimmunologischen oder immunochemischen Nachweisverfahrens zum Nachweis der durch die Applikation des mindestens einen Immunogens gebildeten spezifischen Antikörper im lebenden Nutztier oder in davon stammenden Produkten vorgesehen.

Dabei kann das Immunogen ein Protein und/oder Peptid oder ein Gemisch aus diesen und/oder ein synthetisch hergestelltes Fragment von Aminosäureteilsequenzen natürlich vorkommender Immunogene sein; das Protein und/oder Peptid kann mit einem Trägerstoff gekoppelt sein. - Die Verwendung eines dem zu markierenden Organismus fremden, aber unschädlichen Immunogens zur biologischen Markierung erlaubt einen überraschend einfachen, kostengünstigen und fälschungssicheren Herkunftsnachweis.

### Beispiele:

Als Proteine, die eine ausgezeichnete spezifische Antikörperbildung stimulieren, können dem Nutztier Immunogene wie "keyhole limpet hemocyanine", "green fluorescent protein" aus Aequoria victoria, inaktive Schlangentoxine sowie Virusproteine verabreicht werden. Daneben können auch natürlich vorkommende Peptide oder Polypeptide wie Hirudin, Pheromonotropin oder Ranalexin appliziert werden. Des weiteren können zur Biomarkierung auch synthetisch hergestellte Fragmente von Aminosäureteilsequenzen natürlich vorkommender Immunogene verwendet werden. Schließlich können als Immunogene auch artifizielle Proteine und Peptide dienen, deren Amminosäuresequenz keinem bisher bekannten Stoff entspricht, sondern ausschließlich zum Zweck der Biomarkierung zusammen- bzw. hergestellt werden.

Der Nachweis der durch die genannten Stoffe gebildeten Antikörper erfolgt durch gängige Nachweisverfahren, wie ELISA, EIA und RIA, vorzugsweise unter Verwendung von Teststäbchen, die bsp. in den Tropfsaft des Fleischs des geschlachteten Nutztiers getaucht werden.

## Patentansprüche

1. Verfahren zum Herkunftsnachweis von Nutztieren, insbesondere von Rindern, Schweinen u. dgl., sowie davon stammenden Produkten, umfassend die folgenden Schritte:
a) Biologische Markierung des lebenden Nutztiers durch Applikation mindestens eines für das Nutztier und den Menschen unschädlichen Immunogens, das weder als Impfstoff noch als Arzneimittel, noch über die Nahrungskette, noch über die Umwelt bei der üblichen Nutztierhaltung in den Organismus des Nutztiers gelangt und gegen das in den Nutztieren natürlicherweise keine Antikörper gebildet werden, und
b) Nachweis der durch die Applikation des mindestens einen Immunogens gebildeten spezifischen Antikörper im lebenden Nutztier oder in davon stammenden Produkten mittels eines enzymimmunologischen oder immunochemischen Nachweisverfahrens.

2. Verfahren nach Anspruch 1, wobei als Immunogen(e) mindestens ein Protein und/oder ein Peptid und/oder ein synthetisch hergestelltes Fragment von Aminosäureteilsequenzen natürlich vorkommender Immunogene verwendet wird/werden.

3. Verfahren nach Anspruch 2, wobei ein mit einem Trägerstoff gekoppeltes Protein und/oder Peptid verwendet wird/werden.

4. Verfahren nach einem der vorhergehenden Ansprüche, wobei das Immunogen derart beschaffen ist, daß die gebildeten spezifischen Antikörper dauerhaft im Organismus des Nutztiers verbleiben und mittels enzymimmunologischer oder immunochemischer Nachweisverfahren nachweisbar sind.

5. Verfahren nach einem der vorhergehenden Ansprüche, wobei das Immunogen dem Nutztier durch Injektion appliziert wird.

6. Verfahren nach einem der vorhergehenden Ansprüche, wobei das Immunogen mittels eines Implantats appliziert wird.

7. Verfahren nach einem der vorhergehenden Ansprüche, wobei das Immunogen über die Schleimhaut, vorzugsweise die Nasenschleimhaut, des Nutztiers appliziert wird.

8. Verfahren nach einem der vorhergehenden Ansprüche, wobei eine Mischung von mehreren verschiedenen Immunogenen appliziert wird.

9. Verfahren nach einem der vorhergehenden Ansprüche, wobei der Tropf-/Pressaft des Fleischs des geschlachteten Nutztiers zum Nachweis der spezifischen Antikörper verwendet wird.

10. Verfahren nach einem der vorhergehenden Ansprüche, wobei die Milch oder andere Körpersekrete zum Nachweis der spezifischen Antikörper verwendet wird.

11. Verfahren nach einem der vorhergehenden Ansprüche, wobei das Blut des Nutztiers zum Nachweis der spezifischen Antikörper verwendet wird.

12. Verfahren nach einem der vorhergehenden Ansprüche, wobei als enzymimmunologisches oder immunochemisches eines oder mehrere der folgenden Nachweisverfahren verwendet wird/werden: Enzyme Linked Immunosorbent Assay (= ELISA), Enzyme Immuno Assay (= EIA) ) und Radio Immuno Assay (= RIA).

13. Verfahren nach einem der vorhergehenden Ansprüche, wobei der enzymimmunologische oder immunochemische Nachweis unter Verwendung von Teststäbchen durchgeführt wird.

14. Verwendung mindestens eines
a) für Nutztiere und den Menschen unschädlichen Immunogens zur biologischen Markierung des lebenden Nutztiers sowie davon stammender Produkte, wobei das Immunogen weder als Impfstoff noch als Arzneimittel, noch über die Nahrungskette bei der üblichen Nutztierhaltung in den Organismus des Nutztiers gelangt und gegen das in den Nutztieren natürlicherweise keine Antikörper gebildet werden, und
b) eines enzymimmunologischen oder immunochemischen Nachweisverfahrens zum Nachweis der durch die Applikation des mindestens einen Immunogens gebildeten spezifischen Antikörper im lebenden Nutztier oder in davon stammenden Produkten.

15. Verwendung nach Anspruch 14, wobei das Immunogen ein Protein und/oder Peptid ist und/oder ein synthetisch hergestelltes Fragment von Aminosäureteilsequenzen natürlich vorkommender Immunogene ist.

16. Verwendung nach Anspruch 14 oder 15, wobei das Antigen derart beschaffen ist, daß die gebildeten spezifischen Antikörper dauerhaft im Organismus des Nutztiers verbleiben und mittels enzymimmunologischer oder immunochemischer Nachweisverfahren nachweisbar sind.

17. Verwendung nach Anspruch 15 oder 16, wobei das Protein und/oder Peptid mit einem Trägerstoff gekoppelt ist.

18. Verwendung nach einem der Ansprüche 14 bis 17, wobei eine Mischung von mehreren verschiedenen Immunogenen verwendet wird.

19. Verwendung nach einem der Ansprüche 14 bis 18, wobei der Tropf-/Pressaft des Fleischs des geschlachteten Nutztiers zum Nachweis der Antikörper verwendet wird.

20. Verwendung nach einem der Ansprüche 14 bis 19, wobei das Blut des Nutztiers zum Nachweis der spezifischen Antikörper verwendet wird.

21. Verwendung nach einem der Ansprüche 14 bis 20, wobei die Milch des Nutztiers oder anderer Körpersekrete zum Nachweis der spezifischen Antikörper verwendet wird.

22. Verwendung nach einem der Ansprüche 14 bis 21, wobei als enzymimmunologisches oder immunochemisches eines oder mehrere der folgenden Nachweisverfahren verwendet wird/werden: Enzyme Linked Immunosorbent Assay (= ELISA), Enzyme Immuno Assay (= EIA) und Radio Immuno Assay (= RIA).

23. Verwendung nach einem der Ansprüche 14 bis 22, wobei zum enzymimmunologischen oder immunochemischen Nachweis Teststäbchen verwendet werden.

## Claims

1. Method for detecting the origin of livestock, in particular of cattle, pigs and the like, and of the products derived therefrom, comprising the following steps:
a) biological marking of the living livestock by administering at least one immunogen which is harmless to the livestock and humans, which in customary livestock management, passes into the organism of the livestock neither as vaccine(s) nor as medicament nor via the food chain nor via the environment and against which no antibodies are formed naturally in the livestock, and
b) detection of the specific antibodies formed by the administration of the at least one immunogen in the living livestock or in products derived therefrom using an enzyme-immunological or immunochemical detection method.

2. Method according to Claim 1, wherein the immunogen(s) used is at least one protein and/or peptide and/or a synthesized fragment of a portion of amino acid sequences of naturally occuring immunogens.

3. Method according to Claim 2, wherein a protein and/or peptide coupled to a matrix is/are used.

4. Method according to any of the preceding claims, wherein the immunogen is of a nature such that the specific antibodies formed remain permanently in the organism of the livestock animal and can be detected by means of enzyme-immunological or immunochemical detection methods.

5. Method according to any of the preceding claims, wherein the immunogen is administered to the livestock animal by injection.

6. Method according to any of the preceding claims, wherein the immunogen is administered by means of an implant.

7. Method according to any of the preceding claims, wherein the immunogen being applied via the mucous membrane, preferably the nasal mucosa, of the livestock animal.

8. Method according to any of the preceding claims, wherein a mixture of several different immunogens is administered.

9. Method according to any of the preceding claims, wherein the meat drip/expressible juice of the slaughtered livestock animal is used for detecting the specific antibodies.

10. Method according to any of the preceding claims, wherein the milk or other body secretions are used for detecting the specific antibodies.

11. Method according to any of the preceding claims, wherein the blood of the livestock animal is used for detecting the specific antibodies.

12. Method according to any of the preceding claims, wherein one or more of the following detection methods are used as enzyme-immunological or immunochemical detection method: enzyme-linked immunosorbent assay (= ELISA), enzyme immunoassay (=EIA) and radio immunoassay (=RIA).

13. Method according to any of the preceding claims, wherein the enzyme-immunological or immunochemical detection is carried out using test sticks.

14. Use of at least one
a) immunogen which is harmless to livestock and humans, for the biological marking of the living livestock and of products derived therefrom, and wherein the immunogen in customary livestock management, passes into the organism of the livestock neither as vaccine nor as medicament nor via the food chain against which no antibodies are formed naturally in the livestock and of
b) an enzyme-immunological or immunochemical detection method for detecting, in the living livestock or in products derived therefrom, the specific antibodies formed by applying the at least one immunogen.

15. Use according to Claim 14, wherein the immunogen is a protein and/or peptide and/or a synthesized fragment of a portion of amino acid sequences of naturally occuring immunogens.

16. Use according to Claim 14 or 15, wherein the immunogen is of a nature such that the specific antibodies formed remain permanently in the organism of the livestock animal and can be detected by means of enzyme-immunological or immunochemical detection methods.

17. Use according to Claim 15 or 16, wherein the protein and/or peptide is/are coupled to a matrix.

18. Use according to any of Claims 14 to 17, wherein a mixture of several different immunogens is used.

19. Use according to any of Claims 14 to 18, wherein the meat drip/expressible juices of the slaughtered livestock animal are used for detecting the antibodies.

20. Use according to any of Claim 14 to 19, wherein the blood of the livestock animal is used for detecting the specific antibodies.

21. Use according to any of Claims 14 to 20, wherein the milk of the livestock animal or other body secretions are used for detecting the specific antibodies.

22. Use according to any of Claims 14 to 21, wherein one or more of the following detection methods are used as enzyme-immunological or immunochemical detection method: enzyme-linked immunosorbent assay (=ELISA), enzyme immunoassay (=EIA) and radio immunoassay (=RIA).

23. Use according to any of Claims 14 to 22, wherein test sticks are used for the enzyme-immunological or immunochemical detection.

## Revendications

1. Procédé pour la détermination de l'origine d'animaux de rapport, en particulier de bovins, porcins et similaires, ainsi que de produits qui en dérivent, comprenant les étapes suivantes:
a) marquage biologique de l'animal de rapport vivant, par administration d'au moins un agent immunogène inoffensif pour l'animal de rapport et l'homme, qui ne parvient dans l'organisme de l'animal de rapport ni sous forme de vaccin(s), ni sous forme de médicament(s), ni par la chaîne alimentaire, ni par l'environnement dans l'élevage usuel de l'animal de rapport, et contre le(s) quel(s) aucun anticorps n'est formé naturellement dans les animaux de rapport, et
b) détection des anticorps spécifiques formés par l'administration de l'au moins un agent immunogène, dans l'animal de rapport vivant ou dans des produits en dérivant, au moyen d'un procédé immunochimique ou immunoenzymatique.

2. Procédé selon la revendication 1, dans lequel on utilise comme agent(s) immunogène(s) au moins une protéine et/ou un peptide et/ou un fragment synthétique de une portion de une séquence des amino-acides des agents immunogens formé naturellement.

3. Procédé selon la revendication 2, dans lequel on utilise une protéine et/ou un peptide couplé(s) à une substance porteuse.

4. Procédé selon l'une des revendications précédentes, dans lequel l'agent immunogène est d'une nature telle que les anticorps spécifiques formés restent durablement dans l'organisme de l'animal de rapport et sont détectables par des procédés immunochimiques ou immunoenzymatiques de détection.

5. Procédé selon l'une des revendications précédentes, dans lequel l'agent immunogène est administré par injection à l'animal de rapport.

6. Procédé selon l'une des revendications précédentes, dans lequel l'agent immunogène est administré au moyen d'un implant.

7. Procédé selon l'une des revendications précédentes, dans lequel l'agent immunogène est administré par la muqueuse, de préférence la muqueuse nasale, de l'animal de rapport.

8. Procédé selon l'une des revendications précédentes, dans lequel on administre un mélange de plusieurs agents immunogènes différents.

9. Procédé selon l'une des revendications précédentes, dans lequel, pour la détection des anticorps spécifiques, on utilise l'exsudat/le suc obtenu par pressage de la viande de l'animal de rapport abattu.

10. Procédé selon l'une des revendications précédentes, dans lequel, pour la détection des anticorps spécifiques, on utilise le lait ou d'autres sécrétions corporelles.

11. Procédé selon l'une des revendications précédentes, dans lequel, pour la détection des anticorps spécifiques, on utilise le sang de l'animal de rapport.

12. Procédé selon l'une des revendications précédentes, dans lequel on utilise comme procédé immunoenzymatique ou immunochimique un ou plusieurs des procédés de détection suivants: le dosage immunoenzymatique utilisant un corps absorbé (=ELISA), le dosage immunoenzymatique (= EIA) et al dosage radioimmunologique (= RIA).

13. Procédé selon l'une des revendications précédentes, dans lequel la détection immunoenzymatique ou immunochimique est effectuée avec utilisation de bâtonnets de test.

14. Utilisation d'au moins un
a) agent immunogène inoffensif pour l'animal de rapport et l'homme, qui ne parvient dans l'organisme de l'animal de rapport ni sous forme de vaccin, ni sous forme de médicament, ni par la chaîne alimentaire, ni par l'environnement dans l'élevage usuel de l'animal de rapport, et contre le(s) quel(s) aucun anticorps n'est formé naturellement dans les animaux de rapport, pour le marquage biologique de l'animal de rapport vivant, ainsi que de produits en dérivant, et
b) d'un procédé de détection immunoenzymatique ou immunochimique pour la détection des anticorps spécifiques formés par l'administration de l'au moins un agent immunogène, dans l'animal de rapport vivant ou dans des produits en dérivant.

15. Utilisation selon la revendication 14, dans laquelle l'agent immunogène est une protéine et/ou un peptide et/ou un fragment synthétique de une portion de une séquence des amino-acides des agents immunogènes formé naturellement.

16. Utilisation selon la revendication 14 ou 15, dans laquelle l'antigène est d'une nature telle que les anticorps spécifiques formés restent durablement dans l'organisme de l'animal de rapport et sont détectables par des procédés de détection immunochimiques ou immunoenzymatiques.

17. Utilisation selon la revendication 15 ou 16, dans laquelle la protéine et/ou le peptide est (sont) couplé(s) une substance porteuse.

18. Utilisation selon l'une des revendications 14 à 17, dans laquelle on utilise un mélange de plusieurs agents immunogènes différents.

19. Utilisation selon l'une des revendications 14 à 18, dans laquelle, pour la détection des anticorps, on utilise l'exsudat/le suc obtenu par pressage de la viande de l'animal de rapport abattu.

20. Utilisation selon l'une des revendications 14 à 19, dans laquelle, pour la détection des anticorps spécifiques, on utilise le sang de l'animal de rapport.

21. Utilisation selon l'une des revendications 14 à 20, dans laquelle, pour la détection des anticorps spécifiques, on utilise le lait de l'animal de rapport ou d'autres sécrétions corporelles.

22. Utilisation selon l'une des revendications 14 à 21, dans laquelle on utilise comme procédé immunoenzymatique ou immunochimique un ou plusieurs des procédés de détection suivants : le dosage immunoenzymatique utilisant un corps absorbé (=ELISA), le dosage immunoenzymatique (=EIA) et le dosage radio immunologigue (=RIA).

23. Utilisation selon l'une des revendications 14 à 22, dans laquelle on utilise des bâtonnets de test pour la détection immunoenzymatique ou immunochimique.
